# FASCICULE DE BREVET EUROPEEN

(11) **EP 3 698 768 B1**
(45) Date de publication et mention de la délivrance du brevet: **22.12.2021**
(21) Numéro de dépôt: 20151336.3
(22) Date de dépôt: 13.01.2020
(51) Int. Cl.: A61K 8/19, A61K 9/00, A61K 9/107, A61K 31/191, A61P 17/02, A61Q 19/00

(54) **COMPOSITION CICATRISANTE SOUS FORME D'ÉMULSION HUILE-DANS-EAU**
NARBENBILDENDE ZUSAMMENSETZUNG IN FORM EINER ÖL-IN-WASSER-EMULSION
HEALING COMPOSITION AS AN OIL-IN-WATER EMULSION

(30) Priorité: 24.01.2019 FR 1900620
(43) Date de publication de la demande: 26.08.2020
(73) Titulaire: Les Laboratoires Brothier, 92000 Nanterre (FR)
(72) Inventeur: VILLAIN, Emeline, 49400 Bagneux (FR); GIRARDIERE, Christian, 75016 Paris (FR); LACK, Stéphane, 49070 Saint Lambert la Potherie (FR)
(74) Mandataire: Gevers & Orès

(56) Documents cités:
- EP-A1- 2 540 305
- FR-A1- 3 042 710
- US-A1- 2014 004 164
- YANG WEI,CUIXIA SUN,LEI DAI,LIKE MAO,FANG YUANANDYANXIANG GAO: "Novel Bilayer Emulsions Costabilized by Zein Colloidal Particles and Propylene Glycol Alginate. 2. Influence of Environmental Stresses on Stability and Rheological Properties", J. AGRIC. FOOD CHEM, vol. 4, no. 67, 20 décembre 2018 (2018-12-20), pages 1209-1221, XP002795167,

## Description

### Domaine technique de l'invention

La présente invention a pour objet une composition cicatrisante sous forme d'émulsion huile-dans-eau. Cette composition est avantageusement utilisée dans le traitement des plaies et permet d'assurer une bonne cicatrisation ainsi qu'un apaisement rapide des douleurs en cas de brûlure de la peau.

### Arrière-plan technique

La peau assure de nombreuses fonctions, en particulier la protection contre les éléments extérieurs. Une brûlure entraîne la rupture de ces fonctions, qui sont rétablies lors de la cicatrisation.

Des compositions cicatrisantes sous forme d'émulsion huile-dans-eau qui comprennent du gluconate de zinc en tant qu'agent cicatrisant sont connues (FR 3 042 710 A1).

Il apparaît ainsi souhaitable de disposer d'autres compositions sous forme d'émulsion huile-dans-eau ayant une action cicatrisante satisfaisante et permettant d'apaiser les douleurs en cas de brûlure, l'émulsion présentant en outre une bonne tenue.

La présente invention vise à atteindre ces objectifs.

### Résumé de l'invention

L'invention a ainsi pour objet une composition cicatrisante sous forme d'émulsion huile-dans-eau.

La composition selon l'invention comprend une phase aqueuse, une phase huileuse comprenant de l'huile végétale, elle comprend en tant qu'agents émulsionnants un polymère thermo-gélifiant soluble dans l'eau et de l'alginate de propylène glycol, et elle comprend en tant qu'agents cicatrisants du gluconate de zinc et du gluconate de manganèse.

La demanderesse a constaté que l'association des agents émulsionnants et des agents cicatrisants spécifiques permettait d'obtenir de manière surprenante une mousse agréable à appliquer, de bonne tenue, avec une bonne activité cicatrisante et apaisant rapidement la douleur. Et ce sans ajout de cotensioactifs et stabilisants usuellement utilisée comme du sodium cocoyl glutamate, les carbomers ou l'acide stéraique.

L'utilisation de la composition est préconisée sur les brûlures superficielles, par exemples domestiques, ou en cas de coup de soleil du premier et du deuxième degré, sur les blessures superficielles de la vie courante, par application directe de la composition sur la zone blessée.

La composition selon l'invention permet d'apaiser rapidement les brûlures. Elle participe également au processus de cicatrisation.

La composition est non invasive, destinée à un usage à court terme et présente un risque faible d'irritation ou de sensibilisation.

La phase aqueuse peut être constituée d'eau et la phase huileuse peut être constituée d'huile végétale. Cette huile végétale peut comprendre de l'huile de soja, de l'huile de tournesol, de l'huile d'olive, de l'huile de germe de blé, ou un mélange de ces huiles. La phase huileuse pourrait être constituée d'un seul type d'huile végétale et par exemple d'huile de soja.

On entend par « polymère thermo-gélifiant » un polymère qui, à faible concentration dans l'eau, se présente sous forme liquide (faible viscosité) à température ambiante et qui gélifie à la température corporelle, le phénomène de thermo-gélification étant parfaitement réversible. Par « température ambiante », on entend une température comprise entre 18 et 25°C.

Le polymère thermo-gélifiant peut être choisi parmi la famille des polyéthers, des copolymères de vinylcaprolactone ou des polyacrylamides.

Les polymères thermo-gélifiants utilisables aux fins de l'invention peuvent comprendre, d'une part, des unités hydrosolubles et, d'autre part, des unités présentant dans l'eau une température inférieure critique de démixtion (appelée en abrégé « LCST » pour « lower critical solution température »). En dessous de la LCST, le polymère est parfaitement soluble dans l'eau, alors qu'au-dessus de cette température les portions à LCST s'associent et perdent leur solubilité dans l'eau, formant ainsi des nœuds de réticulation entre les chaînes polymères. Le polymère s'apparente alors à un réseau tridimensionnel, ce qui conduit à l'obtention d'un gel. Cette association de chaînes à LCST au sein des micro-domaines hydrophobes au-delà de la température de démixtion étant de nature physique, le phénomène de gélification est alors parfaitement réversible. Cette propriété de gélification avec la température n'est possible que si la concentration en polymère est supérieure à la concentration micellaire critique pour permettre des interactions entre les unités à LCST portées par des chaînes polymères différentes.

Les polymères utilisés dans l'invention peuvent être aussi bien des polymères à blocs ou des polymères greffés qui comprennent, d'une part, des unités hydrosolubles et d'autre part des unités à LCST. Ces unités hydrosolubles peuvent être des polymères d'origine naturelle ou d'origine synthétique obtenus par polymérisation en chaîne ou par polycondensation.

Les unités à LCST seront préférentiellement choisies parmi :
- les polyéthers tels que le poly(oxyde de propylène) (POP) et ses copolymères statistiques et à blocs avec le poly(oxyde d'éthylène) (POE),
- les dérivés N-substitués de l'acrylamide comme le poly(N-isopropylacrylamide) ou le poly(N-éthylacrylamide),
- le polyvinylcaprolactame et les copolymères de vinylcaprolactame.

Les polymères thermogélifiants susceptibles de convenir à l'invention peuvent être, par exemple, choisis parmi ceux décrits dans les demandes de brevets et brevets suivants : FR2694939, FR2788008, FR2820976, FR2856923, GB2408510, EP0629649, EP1307501, EP1407791, WO97/00275, WO98/06438, WO98/29487, WO98/48768, WO98/50005, WO00/00222, WO00/07603, WO00/35961, WO00/38851, WO01/41735, WO02/032560, WO02/076392, WO03/008462, WO03/106536, WO04/006872, US2003/0099709, US6645476, US6689855, US6689856, US6870012 et US6878754.

Les polymères thermogélifiants particulièrement adaptés sont les polyuréthanes comportant des groupements poly(oxyde d'éthylène-b-oxyde de propylène-b-oxyde d'éthylène) (POE-b-POP-b-POE) tels que ceux décrits dans les brevets et les demandes de brevets FR2840907, EP692506, EP1407791, WO 03 106536, US 7339013.

Ces polyuréthanes sont obtenus par polycondensation de diisocyanates et de diols triblocs POE-b-POP-b-POE thermosensibles en milieu non anhydre et peuvent comprendre des groupements urée et/ou allophanates. Ces polymères, connus sous le nom d'ExpertGel^{®} et commercialisés par la société PolymerExpert, présentent de nombreux avantages, en particulier: une faible concentration de polymères est nécessaire pour une augmentation importante de la viscosité en fonction de la température, ils présentent des propriétés lubrifiantes importantes, ils sont muco-adhésifs, - ils présentent le même comportement thermogélifiant dans une gamme de pH allant de 1 à 12, ils sont compatibles avec les sels, tensio-actifs et la plupart des excipients pharmaceutiques et cosmétiques.

A titre d'exemple, on peut citer le polymère thermo-gélifiant EG230, qui est un polyuréthane ramifié contenant des unités POE-b-POP-b-POE, dont la désignation INCI (International Nomenclature of Cosmetic Ingrédients) en langue anglaise est Bis-Methoxy PEG-13 PEG-502/PPG-57 SMDI Copolymer.

L'acide alginique et ses dérivés (base conjuguée, sels et esters) les alginates sont des polysaccharides principalement obtenus à partir d'une famille d'algues brunes : les laminaires ou les fucus.

L'alginate est le nom commun pour une famille de polymères formés de deux monomères liés ensemble : le mannuronate ou acide mannuronique et le guluronate ou acide guluronique.

La formule générale des alginates est la suivante :

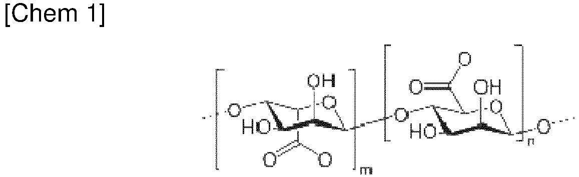

La proportion et la distribution de ces deux monomères sont déterminantes pour une large expansion des propriétés physiques et chimiques de l'alginate, on parle ainsi de rapport mannuronique gluronique ou encore de rapport M/G. Sa composition chimique varie selon les diverses espèces d'algues, les différentes parties de la même plante et est sujette aux changements saisonniers. Néanmoins, par sélection de matières premières aux différentes propriétés, il est possible de fabriquer une variété d'alginate aux caractéristiques constantes.

Les produits à base d'alginate ont des propriétés intrinsèques, naturelles, très efficaces contre le traitement des plaies. Ces produits sont en particulier destinés à l'hémostase et à la cicatrisation.

L'alginate de propylène glycol peut être préparé par réaction de l'acide alginique avec de l'oxyde de propylène.

La composition peut comprendre en outre du chlorure de benzalkonium, ce qui permet une formulation avec une biocharge contrôlée voir une formulation stérile.

La teneur en phase aqueuse peut être comprise entre 50 et 80 % du poids total de la composition, et de préférence entre 60 et 68 % du poids total de la composition.

La teneur en phase huileuse peut être comprise entre 20 et 50 % du poids total de la composition, et de préférence entre 30 et 40 % du poids total de la composition.

La teneur en polymère thermo-gélifiant soluble dans l'eau peut être comprise entre 1 et 5 % du poids total de la composition, et de préférence entre 1,5 et 3,5 % du poids total de la composition.

La teneur en alginate de propylène glycol peut être comprise entre 0,5 et 2 % du poids total de la composition, et de préférence entre 0,6 et 1 % du poids total de la composition.

La teneur en gluconate de zinc peut être comprise entre 0,001 et 0,03 % du poids total de la composition, et de préférence entre 0,01 et 0,02 % du poids total de la composition.

La teneur en gluconate de manganèse peut être comprise entre 0,01 et 0,5 % du poids total de la composition, et de préférence entre 0,03 et 0,1% du poids total de la composition.

La teneur en chlorure de benzalkonium peut être comprise entre 0,1 et 0,6 % du poids total de la composition, et de préférence entre 0,2 et 0,4 % du poids total de la composition.

La composition peut également comprendre des vitamines notamment la vitamine A, la vitamine C et la vitamine B5. Elle peut également comprendre du glycérophosphate de calcium ou de lactate de calcium ou encore du sorbate de potassium. La vitamine A stimule la réponse immunitaire, la libération des facteurs de croissance, la multiplication, la différenciation des fibroblastes et la synthèse du collagène. De larges doses de vitamine A améliorent la cicatrisation et la réponse inflammatoire. La vitamine C agit dans les processus de cicatrisation par la mobilisation d'éléments : Fe, Cu, Zn, Mn, Mg, elle augmente le taux de fibroblastes et a un effet positif et sur le processus de cicatrisation avec une cicatrice plus solide et résistante. La vitamine B5 accélère la fermeture des plaies et augmente la résistance des cicatrices. Le glycérophosphate de calcium et le lactate de calcium permettent d'accélérer la cicatrisation grâce à l'activité du calcium notamment. Le sorbate de potassium est un conservateur aux propriétés antifongique et antibactérienne connues.

La composition peut se présenter sous différentes formes ou conditionnements, et notamment sous la forme d'un aérosol, d'une ampoule, notamment à fond souple, d'une enveloppe munie d'une zone sécable, d'un tube, ou encore d'un flacon.

L'invention a également pour objet une composition décrite ci-dessus pour une utilisation cicatrisante et/ou apaisante.

L'invention est illustrée de manière non limitative par l'exemple qui suit. Les pourcentages sont exprimés en poids.

### Exemple

Préparation d'une composition cicatrisante et apaisante pouvant être déposée sous forme d'aérosol sur la peau

**[Tableau 1]**

| **Composants** | **% en poids** |
|---|---|
| Eau déminéralisée | 66,84 |
| Huile végétale (ex. soja) | 30 |
| EG230 | 2 |
| PGA | 0,8 |
| Gluconate de zinc | 0,0125 |
| Gluconate de manganèse | 0,05 |
| Chlorure de benzalkonium | 0,3 |

La composition est préparée en deux étapes de la manière suivante. Dans un premier temps, la phase aqueuse est préparée. Elle comprend EG230, PGA, gluconate de zinc et de manganèse, et chlorure de benzalkonium dissous dans de l'eau déminéralisée. Les composants sont ainsi ajoutés un par un dans l'eau selon l'ordre suivant : EG230, PGA, gluconate de zinc, gluconate de manganèse et chlorure de benzalkonium, le tout sous agitation à 1500 tr/min pendant 45 minutes. Dans un deuxième temps, l'huile (par exemple de soja, mais qui pourrait être n'importe quelle autre huile végétale : tournesol, olive, germe de blé, ou un mélange d'huiles végétales) est ajoutée sous agitation plus vigoureuse à 2000 tr/min, pendant 1 minute puis à 1500tr/min pendant 15 minutes. Lorsque l'émulsion est obtenue, elle est conditionnée sous pression en présence de gaz qui permettront la formation d'une mousse, comme par exemple un mélange de gaz butane/propane.

Le polymère ExpertGel^{®} 230 ou EG230, est un polymère semi-cristallin thermo-gélifiant. Il est filmogène, non irritant, avec un pH compris entre 6 et 8 et est donc compatible avec le pH de la peau. Il permet de stabiliser la composition.

Le PGA, propylène glycol alginate, est un ester d'alginate qui est utilisé pour ses propriétés stabilisantes et émulsifiantes. En utilisation conjointe avec EG230, il permet d'augmenter la stabilité de la composition notamment après incorporation de gaz dans cette dernière.

Le zinc a une activité connue et reconnue dans la cicatrisation notamment par son activation des macrophages (cellules clés de la réparation tissulaire) ; sa participation à la constitution de la matrice extracellulaire et son activité antiradicalaire qui protège les cellules.

Le manganèse favorise également la cicatrisation en améliorant l'adhésion cellulaire au fibrogène et la maturation du collagène de la matrice extracellulaire.

Le chlorure de benzalkonium apporte une composante bactéricide avec un spectre d'activité dit large (sur bactérie gram+ et gram-) et ce à des concentrations faibles.

## Revendications

1. Composition cicatrisante sous forme d'émulsion huile-dans-eau, **caractérisée en ce qu'**elle comprend une phase aqueuse, une phase huileuse comprenant de l'huile végétale, **en ce qu'**elle comprend en tant qu'agents émulsionnants un polymère thermo-gélifiant soluble dans l'eau et de l'alginate de propylène glycol, et **en ce qu'**elle comprend en tant qu'agents cicatrisants du gluconate de zinc et du gluconate de manganèse.

2. Composition selon la revendication 1, **caractérisée en ce que** la phase huileuse comprend de l'huile de soja, de l'huile de tournesol, de l'huile d'olive, de l'huile de germe de blé, ou un mélange de ces huiles.

3. Composition selon la revendication 1 ou 2, **caractérisée en ce que** la phase huileuse est constituée d'huile de soja.

4. Composition selon l'une des revendications 1 à 3, **caractérisée en ce que** le polymère thermo-gélifiant soluble dans l'eau est choisi parmi les polyuréthanes comportant des groupements poly(oxyde d'éthylène-b-oxyde de propylène-b-oxyde d'éthylène).

5. Composition selon l'une des revendications 1 à 4, **caractérisée en ce qu'**elle comprend en outre du chlorure de benzalkonium.

6. Composition selon l'une des revendications 1 à 5, **caractérisée en ce que** la teneur en phase aqueuse est comprise entre 50 et 80 % du poids total de la composition, et de préférence entre 60 et 68 % du poids total de la composition.

7. Composition selon l'une des revendications 1 à 6, **caractérisée en ce que** la teneur en phase huileuse est comprise entre 20 et 50 % du poids total de la composition, et de préférence entre 30 et 40 % du poids total de la composition.

8. Composition selon l'une des revendications 1 à 7, **caractérisée en ce que** la teneur en polymère thermo-gélifiant soluble dans l'eau est comprise entre 1 et 5 % du poids total de la composition, et de préférence entre 1,5 et 3,5 % du poids total de la composition.

9. Composition selon l'une des revendications 1 à 8, **caractérisée en ce que** la teneur en alginate de propylène glycol est comprise entre 0,5 et 2 % du poids total de la composition, et de préférence entre 0,6 et 1 % du poids total de la composition.

10. Composition selon l'une des revendications 1 à 9, **caractérisée en ce que** la teneur en gluconate de zinc est comprise entre 0,001 et 0,03 % du poids total de la composition, et de préférence entre 0,01 et 0,02 % du poids total de la composition.

11. Composition selon l'une des revendications 1 à 10, **caractérisée en ce que** la teneur en gluconate de manganèse est comprise entre 0,01 et 0,5 % du poids total de la composition, et de préférence entre 0,03 et 0,1 % du poids total de la composition.

12. Composition selon l'une des revendications 1 à 11, **caractérisée en ce qu'**elle se présente sous la forme d'un aérosol, d'une ampoule, notamment à fond souple, d'une enveloppe munie d'une zone sécable, d'un tube, ou d'un flacon.

13. Composition selon l'une des revendications 1 à 12 pour une utilisation hémostatique, cicatrisante, et/ou apaisante.

## Patentansprüche

1. Wundheilungszusammensetzung in Form einer Öl-in-Wasser-Emulsion, **dadurch gekennzeichnet, dass** sie eine wässrige Phase, eine ölige Phase, die Pflanzenöl umfasst, umfasst, dadurch, dass sie als Emulgiermittel ein wasserlösliches thermogelierendes Polymer und Propylenglycolalginat umfasst, und dadurch, dass sie als Wundheilungsmittel Zinkgluconat und Mangangluconat umfasst.

2. Zusammensetzung nach Anspruch 1, **dadurch gekennzeichnet, dass** die ölige Phase Sojaöl, Sonnenblumenöl, Olivenöl, Weizenkeimöl oder eine Mischung dieser Öle umfasst.

3. Zusammensetzung nach Anspruch 1 oder 2, **dadurch gekennzeichnet, dass** die ölige Phase aus Sojaöl besteht.

4. Zusammensetzung nach einem der Ansprüche 1 bis 3, **dadurch gekennzeichnet, dass** das wasserlösliche thermogelierende Polymer ausgewählt ist aus Polyurethanen, die Poly(ethylenoxid-b-propylenoxid-b-ethylenoxid)-Gruppen beinhalten.

5. Zusammensetzung nach einem der Ansprüche 1 bis 4, **dadurch gekennzeichnet, dass** sie des Weiteren Benzalkoniumchlorid umfasst.

6. Zusammensetzung nach einem der Ansprüche 1 bis 5, **dadurch gekennzeichnet, dass** der Gehalt an wässriger Phase zwischen 50 und 80 % des Gesamtgewichts der Zusammensetzung und vorzugsweise zwischen 60 und 68 % des Gesamtgewichts der Zusammensetzung liegt.

7. Zusammensetzung nach einem der Ansprüche 1 bis 6, **dadurch gekennzeichnet, dass** der Gehalt an öliger Phase zwischen 20 und 50% des Gesamtgewichts der Zusammensetzung und vorzugsweise zwischen 30 und 40% des Gesamtgewichts der Zusammensetzung liegt.

8. Zusammensetzung nach einem der Ansprüche 1 bis 7, **dadurch gekennzeichnet, dass** der Gehalt an wasserlöslichem thermogelierenden Polymer zwischen 1 und 5 % des Gesamtgewichts der Zusammensetzung und vorzugsweise zwischen 1,5 und 3,5 % des Gesamtgewichts der Zusammensetzung liegt.

9. Zusammensetzung nach einem der Ansprüche 1 bis 8, **dadurch gekennzeichnet, dass** der Gehalt an Propylenglycolalginat zwischen 0,5 und 2 % des Gesamtgewichts der Zusammensetzung und vorzugsweise zwischen 0,6 und 1 % des Gesamtgewichts der Zusammensetzung liegt.

10. Zusammensetzung nach einem der Ansprüche 1 bis 9, **dadurch gekennzeichnet, dass** der Gehalt an Zinkgluconat zwischen 0,001 und 0,03 % des Gesamtgewichts der Zusammensetzung und vorzugsweise zwischen 0,01 und 0,02 % des Gesamtgewichts der Zusammensetzung liegt.

11. Zusammensetzung nach einem der Ansprüche 1 bis 10, **dadurch gekennzeichnet, dass** der Gehalt an Mangangluconat zwischen 0,01 und 0,5 % des Gesamtgewichts der Zusammensetzung und vorzugsweise zwischen 0,03 und 0,1 % des Gesamtgewichts der Zusammensetzung liegt.

12. Zusammensetzung nach einem der Ansprüche 1 bis 11, **dadurch gekennzeichnet, dass** sie in Form eines Aerosol, einer Ampulle, insbesondere mit nachgiebigem Boden, einer mit einer trennbaren Zone ausgestatteten Hülle, einer Tube oder eines Flakons vorliegt.

13. Zusammensetzung nach einem der Ansprüche 1 bis 12 für eine hämostatische, wundheilende und/oder beruhigende Anwendung.

## Claims

1. A healing composition in the form of an oil-in-water emulsion, **characterized in that** it comprises an aqueous phase, an oil phase comprising vegetable oil, **in that** it comprises as emulsifying agents a water-soluble thermo-gelling polymer and propylene glycol alginate, and **in that** it comprises as healing agents zinc gluconate and manganese gluconate.

2. The composition according to claim 1, **characterized in that** the oil phase comprises soybean oil, sunflower oil, olive oil, wheat germ oil, or a mixture of these oils.

3. The composition according to claim 1 or 2, **characterized in that** the oil phase consists of soybean oil.

4. The composition according to one of claims 1 to 3, **characterized in that** the water-soluble thermo-gelling polymer is selected from polyurethanes comprising poly(ethylene oxide-b-propylene oxide-b-ethylene oxide) groups.

5. The composition according to one of claims 1 to 4, **characterized in that** it further comprises benzalkonium chloride.

6. The composition according to one of claims 1 to 5, **characterized in that** the aqueous phase content is between 50 and 80% of the total weight of the composition, and preferably between 60 and 68% of the total weight of the composition.

7. The composition according to one of claims 1 to 6, **characterized in that** the oil phase content is between 20 and 50% of the total weight of the composition, and preferably between 30 and 40% of the total weight of the composition.

8. The composition according to one of claims 1 to 7, **characterized in that** the content of water-soluble thermo-gelling polymer is between 1 and 5% of the total weight of the composition, and preferably between 1.5 and 3.5% of the total weight of the composition.

9. The composition according to one of claims 1 to 8, **characterized in that** the content of propylene glycol alginate is between 0.5 and 2% of the total weight of the composition, and preferably between 0.6 and 1% of the total weight of the composition.

10. The composition according to one of claims 1 to 9, **characterized in that** the content of zinc gluconate is between 0.001 and 0.03% of the total weight of the composition, and preferably between 0.01 and 0.02% of the total weight of the composition.

11. The composition according to one of claims 1 to 10, **characterized in that** the manganese gluconate content is between 0.01 and 0.5% of the total weight of the composition, and preferably between 0.03 and 0.1% of the total weight of the composition.

12. The composition according to one of claims 1 to 11, **characterized in that** it is in the form of an aerosol, of an ampoule, in particular with a flexible bottom, of an envelope provided with a breakable zone, of a tube or of a bottle.

13. The composition according to one of claims 1 to 12 for haemostatic, healing and/or soothing use.
